(19) Europäisches Patentamt / European Patent Office / Office européen des brevets

(11) **EP 4 421 729 B1**

(12) **EUROPEAN PATENT SPECIFICATION**

(45) Date of publication and mention
of the grant of the patent:
**18.03.2026  Bulletin 2026/12**

(21) Application number: **23837125.6**

(22) Date of filing: **19.09.2023**

(51) International Patent Classification (IPC):
*G06T 5/00* (2024.01)        *G06T 11/00* (2026.01)
*G06T 7/254* (2017.01)       *A61B 6/00* (2024.01)
*G06T 5/50* (2006.01)        *G06T 5/70* (2024.01)

(52) Cooperative Patent Classification (CPC):
**A61B 6/4405; A61B 6/00; A61B 6/4441;**
**A61B 6/5258; G06T 5/50; G06T 5/70; G06T 12/10;**
G06T 2207/10016; G06T 2207/10116;
G06T 2207/10121; G06T 2207/20182;
G06T 2207/30004; G06T 2211/412

(86) International application number:
**PCT/KR2023/014198**

(87) International publication number:
**WO 2024/143776 (04.07.2024 Gazette 2024/27)**

(54) **IMAGE PROCESSING METHOD AND APPARATUS FOR REDUCING NOISE INCLUDED IN IMAGE ACQUIRED BY RADIOGRAPHY**

BILDVERARBEITUNGSVERFAHREN UND -VORRICHTUNG ZUR RAUSCHVERMINDERUNG IN EINEM DURCH RADIOGRAPHIE AUFGENOMMENEN BILD

PROCÉDÉ ET APPAREIL DE TRAITEMENT D'IMAGE PERMETTANT DE RÉDUIRE UN BRUIT INTÉGRÉ DANS UNE IMAGE ACQUISE PAR RADIOGRAPHIE

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**

(30) Priority: **26.12.2022  KR 20220184659**

(43) Date of publication of application:
**28.08.2024  Bulletin 2024/35**

(73) Proprietor: **DRTECH CORP**
**Seongnam-Si, Gyeonggi-do 13216 (KR)**

(72) Inventors:
• **SHIN, Choul Woo**
**Seongnam-si**
**Gyeonggi-do 13476 (KR)**
• **PARK, June Kyu**
**Seoul 05613 (KR)**
• **YU, Young Gi**
**Gwacheon-si**
**Gyeonggi-do 13835 (KR)**

(74) Representative: **Manitz Finsterwald**
**Patent- und Rechtsanwaltspartnerschaft mbB**
**Martin-Greif-Straße 1**
**80336 München (DE)**

(56) References cited:
CN-B- 102 074 022        JP-A- 2002 539 870
JP-A- 2008 113 330        KR-B1- 101 432 864
KR-B1- 101 535 010        KR-B1- 102 448 069
KR-B1- 102 448 069        US-B2- 8 655 034

EP 4 421 729 B1

**Description**

[Technical Field]

This invention relates to a method and device for image processing to reduce noise included in images acquired by radiation imaging.

[Background Art]

Various medical diagnoses and treatments are carried out based on images taken with radiation imaging devices that utilize radiation like X-rays. Typically, medical images obtained using X-rays not only contain anatomical information of a subject, i.e., a human body, but also noises generated due to imaging environments and the performance of an apparatus. Such noises degrade images, impairing the ability to anatomically interpret a patient's condition. The degree of image degradation is determined by various factors; for example, degradation of images due to noise inherent in a sensor itself, and degradation caused by noise arising from varying X-ray doses.

On the other hand, long exposure to radiation during X-ray imaging can lead to adverse effects due to radiation exposure. For this reason, there's a demand to obtain X-ray images by irradiating with low-dose X-rays to minimize radiation exposure. However, when imaging with low-dose X-rays, the photon density of the incoming X-rays decreases, leading to a significant increase in the concentration of quantum mottle, which degrades the quality of the image.

For these reasons, various methods have been introduced to effectively remove noise from images acquired using low-dose X-rays. For instance, in Korean Patent Registration No. 10-1432864, a noise reduction technique through recursive filtering was introduced, in which a noise component image is acquired by thresholding and downsizing a difference map obtained by the difference between the current frame and the previous frame and this noise component is then added to the current frame to create an output image of the current frame, and the noise-reduced output image is stored in memory. Related technology is also known from KR 102 448 069 B1.

However, the noise reduction technique using recursive filtering is highly effective in eliminating line noise and being applied for stationary subjects without motion, but its noise-reduction performance decreases as the subject's motion increases and in particular, when the motion of the subject is misinterpreted, it leads to problems of degrading image quality due to motion blur. Therefore, setting a threshold value in the recursive filtering method to discriminate motion and noise from the difference map of the current and previous frames is crucial. In the aforementioned Korean Patent Registration No. 10-1432864, if the absolute value of each pixel in the difference map exceeds a predetermined threshold greater than the standard deviation of the noise, it is determined that there is motion in the subject; otherwise, it is determined that there's no motion. However, given the nature of X-ray images, factors such as dose conditions during imaging, density of a subject, and the degree of motion of a subject can cause significant variations in the intensity and variance of the noise, and thus the range of the threshold value also increases so that it is challenging to set an appropriate threshold. Additionally, when there is a motion in a subject, the effectiveness of the recursive filtering is suppressed, resulting in decreased noise reduction efficiency.

<Prior Documents>

-    Korean Patent No. 10-1432864 (2014.08.26.)
-    Japanese Patent No. 6744440 (2020.08.19.)

[Detailed Description of the Invention]

[Technical Problem]

The object of this invention is to provide a solution that effectively reduces noise by utilizing a cumulative motion map, which accumulates images over time after noise reduction processing based on motion comparison in a difference map.

[Technical Solutions]

The present invention is set out in the appended claims. An image processing device according to an embodiment of the present invention performs noise reduction processing on images obtained by radiation imaging using a recursive way. The image processing device includes: an image input unit that receives frame-by-frame images obtained by the radiation imaging; a motion map generation unit that generates a motion map having motion detection information for each pixel of a difference map obtained by a difference between a current frame image and a previous frame image; a cumulative motion map generation unit that generates a cumulative motion map based on the generated motion map and a cumulative motion map up to a previous frame; a statistical value map generation unit that generates a statistical value map formed by assigning a statistical value representing a motion probability of each pixel of the cumulative motion map; and an output image generation unit that generates an output image of a current frame by mixing the current frame image and the output image of the previous frame based on the cumulative motion map.

The motion map generation unit includes: a difference map generation unit that generates the difference map based on the difference between the current frame image and the previous frame image; and a threshold processing unit that generates the motion map having the motion detection information for each pixel through a threshold processing of pixel values of the difference map.

The threshold processing unit may be configured to perform the threshold processing of the difference map using an adaptive threshold, the size of which varies depending on the magnitude of the statistical value of the statistical value map.

The threshold processing unit is configured to set a threshold value of each pixel for the threshold processing lower as a motion probability indicated by the statistical value of a pixel corresponding to the statistical map is higher.

The statistical value map generation unit is configured to calculate the statistical value based on a pixel mask that comprises a plurality of pixels comprising each pixel of the cumulative motion map and assigns the calculated statistical value to each pixel to generate the statistical value map.

The statistical value is an entropy, a variance, or a standard deviation of the pixel values of the plurality of pixels in the pixel mask.

The difference map may be obtained using an image of the current frame and one or more images of the previous frame.

The previous frame image may be an output image of the previous frame.

The cumulative motion map generation unit may be configured to update the cumulative motion map using the statistical value map.

The output image generation unit may be configured to determine a mixing ratio of the current frame image and the output image of the previous frame based on the updated cumulative motion map.

According to an embodiment of the present invention, an image processing method for processing an image obtained by radiation imaging for reducing noise through a recursive way includes: receiving frame-by-frame images obtained through the radiation imaging; generating a motion map that includes motion detection information for each pixel of a difference map obtained by a difference between a current frame image and a previous frame image; generating a cumulative motion map based on the generated motion map and the motion map accumulated up to a previous frame; generating a statistical value map formed by assigning statistical values representing a motion probability of a plurality of pixels including each pixel of the cumulative motion map; and mixing the current frame image and an output image of the previous frame based on the cumulative motion map to produce an output image of the current frame.

In the generating of the motion map, a threshold of each pixel for the thresholding is set lower as a motion probability indicated by the statistical value of the corresponding pixel of the statistical value map increases.

In the generating of the statistical value map, the statistical value map is generated by calculating the statistical value based on a pixel mask which comprises a plurality of pixels having each pixel of the cumulative motion map and then assigning the calculated statistical value to each pixel.

The statistical value is an entropy, a variance, or a standard deviation of pixel values of the plurality of pixels of the pixel mask.

The generating of the output image may include: updating the cumulative motion map using the statistical value map; and determining a mixing ratio of the current frame image and the output image of the previous frame based on the updated cumulative motion map.

In the updating of the cumulative motion map, a threshold processing may be performed on the statistical value map and pixel values of the pixels of the cumulative motion map may be selectively increased or decreased using the threshold-processed statistical value map, thereby updating the cumulative motion map.

The difference map may be obtained by a difference between the current frame image and an output image of the previous frame.

An imaging processing method according to another embodiment of the present invention may further include updating the cumulative motion map using the statistical value map.

In the generating of the output image, a mixing ratio of the current frame image and the output image of the previous frame may be determined based on the updated cumulative motion map.

[Effects of the Invention]

According to the present invention, an image with reduced noise can be generated through a cumulative motion map.

[Brief Description of the Drawings]

FIG. 1 is a schematic diagram illustrating an example of a radiation imaging device in the form of a C-arm, to which an image processing device according to an embodiment of the present invention is applied.
FIG. 2 is a block diagram of an image processing device according to an embodiment of the present invention.
FIG. 3 is a flowchart of an image processing method according to an embodiment of the present invention.
FIG. 4 is a graph comparatively showing the distribution of pixel values in a difference map of the original signal and the distribution of pixel values in a difference map obtained after Anscombe transformation for the same input images (previous frame and current frame) of a stationary object.

FIG. 5 is a schematic diagram illustrating an algorithm of an image processing method according to an embodiment of the present invention.

FIG. 6 shows an example of a motion map outputted when a hand phantom is moved from the right to the left in the previous frame image and the current frame image.

FIG. 7 is a drawing illustrating a method of creating a cumulative motion map using a motion map.

FIG. 8 is a drawing illustrating the generation of a motion map and a cumulative motion map based on an input image as a frame progresses.

FIG. 9 exemplarily shows a motion map and an output image obtained in a situation where a thin lead with a bar shape passes over a stationary pelvic phantom.

FIG. 10 exemplarily shows the motion maps under the same conditions as FIG. 9, but with the threshold for motion detection raised and lowered.

FIG. 11 exemplarily shows an entropy map created by calculating an entropy through pixel masking calculations in the cumulative motion map.

FIG. 12 is a diagram showing a method for generating a motion map according to another embodiment of the present invention.

FIG. 13 is a schematic diagram showing an algorithm of an image processing method according to another embodiment of the present invention.

FIG. 14 is a diagram showing a process of updating a cumulative motion map using a threshold-processed entropy map in an image processing method of FIG. 13.

[Detailed Description of Embodiment of the Invention]

Referring to the attached drawings below, embodiments of the present invention are described in detail so that a person having ordinary skill in the technical field to which the present invention pertains can easily carry them out. However, the present invention can be implemented in various different forms and is not limited to the embodiments described.

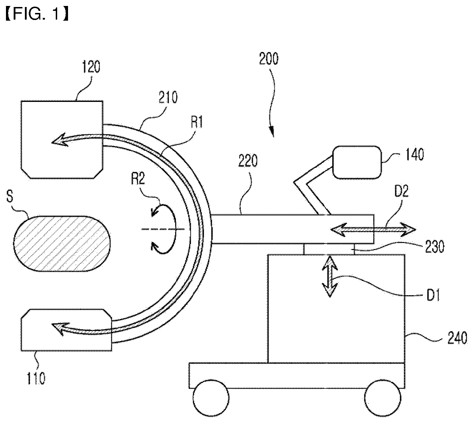

In FIG. 1, an example of a C-arm type X-ray imaging device with an image processing device having noise reduction functions according to an embodiment of the present invention is shown. The image processing device according to an embodiment of the present invention can be applied as part of an imaging device, as exemplified in FIG. 1, and can also be configured as a separate device from the imaging device to perform image processing functions.

The imaging device to which the image processing device according to an embodiment of the present invention can be applied may be configured as a C-arm type, as shown in FIG. 1, and can be composed of a photographing device capable of acquiring video. For example, it can be configured to capture a region of interest in a subject (S) using radiation such as X-rays.

Referring to FIG. 1, a radiation imaging device can be equipped with a radiation irradiation part 110 that emits radiation, for example, X-rays, and an image acquisition part 120 that detects radiation that has passed through a subject S and acquires image data. The radiation irradiation part 110 and the image acquisition part 120 can be supported on both ends of a C-arm 210. For instance, the radiation imaging device can be applied to devices such as a mobile C-arm X-ray imaging device, an interventional X-ray device, or an interventional angiography C-arm X-ray device, or the like.

A support structure 200 supports the radiation irradiation part 110 and the image acquisition part 120 and is configured to allow changes in a spatial position and rotation position of the radiation irradiation part 110 and the image acquisition part 120 for changing of such as the imaging position and angle of the subject S. For example, the support structure 200 may include a support body 240, a lift column 230 attached to the support body 240 to be movable in a vertical direction D1, and a forward-backward moving arm 220 that can move in the vertical direction with the lift column 230 and can move relative to the lift column 230 in a horizontal direction D2.

The C-arm 210 is coupled to the forward and backward moving arm 220 so as to be relatively rotatable in at least one rotation direction with respect to the forward and backward moving arm 220, and the radiation irradiation part 110 and the image acquisition part 120 are respectively connected to both ends of the C-arm 210. In this regard, the C-arm 210 is connected to the forward and backward moving arm 220 to be relatively rotatable with respect to the forward and backward moving arm 220 in at least one rotation direction, for example in an orbital rotation direction R1 and an axis rotation direction R2 centered on the direction parallel to the horizontal direction of the forward and backward moving arm 220, while being able to move up and down in the vertical direction and to move forward and backward in the horizontal direction together with the forward and backward arm 220. Although not shown in the drawing, the support structure 200 may include actuators such as a motor for upward and downward movement of the lift column 230, the horizontal movement of the forward and backward moving arm 220, and the rotation of the C-arm 210. Elements for supporting and driving the C-arm 210, which is a support member supporting the radiation irradiation unit 110 and the image acquisition unit 120, namely the forward and backward moving arm 220, the lift column 230, and the actuators provided thereto, may be referred to as driving elements for driving the C-arm 210, and the combinations thereof may be referred to as a driving part. Additionally, it may be configured to enable a panning rotation of the C-arm 210 through horizontal rotation of the forward and backward moving arm 220. The shape of the support member is not limited to the C-shape, and in another embodiment of the present invention, an arm having a U-shape, a G-shape, etc. may be used as a support member instead of the C-shaped arm.

A display unit 140 is configured to display at least one of real-time location information, image data, reference location information, and radiation output information. The display unit 140 can be any device capable of displaying information and images. For example, it can be a printer, a CRT display, an LCD display, a PDP display, an OLED display, an FED display, an LED display, a DLP display, a PFD display, a 3D display, a transparent display, and so on. Additionally, the display unit 140 can also be implemented in a form capable of displaying information and receiving input, such as a touchscreen.

The image processing device 10 according to an embodiment of the present invention can be implemented in the form of an information processing device, such as one or more computers, capable of information processing and computation. For instance, the computer may include control means like a CPU, storage means like ROM (read-only memory) or RAM (random access memory), and graphic control means like a GPU (graphics processing unit). The computer can also include communication means like a network card, and input/output means like a keyboard, display, or touchscreen. Components of such a computer can be connected via a bus as known in the art and can be operated and controlled by the execution of a program stored in the storage means.

The image processing device 10, which can be implemented in the form of an information-processing computer, can be installed in the radiation imaging device depicted in FIG. 1 to perform image processing functions. In this case, it can be configured as part of the radiation imaging device to receive the captured image, process it, and display the processed image on the imaging display means of the radiation imaging device.

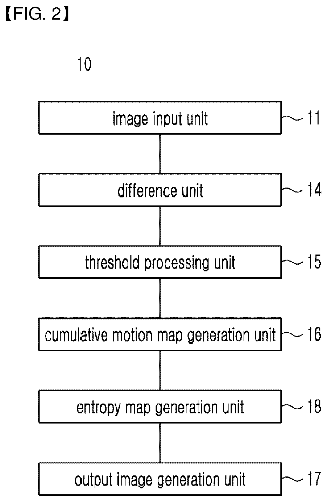

Referring to FIG. 2, the image processing device 10 according to an embodiment of the present invention includes multiple functional configurations for noise reduction processing, namely, an image input unit 11, a difference unit 14, a threshold processing unit 15, a cumulative motion map generation unit 16, and an output image generation unit 17. In this regard, a motion map is produced by the consecutive operations of the difference unit 14 and the threshold processing unit 15. In this sense, the difference unit 14 and the threshold processing unit 15 may be regarded as a motion map generation unit.

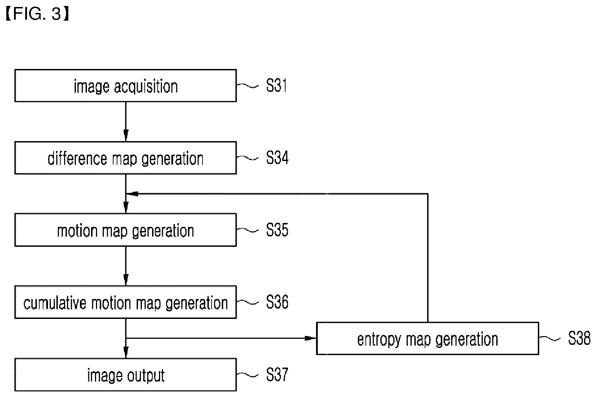

Referring to Fig. 3, an image processing method according to one embodiment of the present invention that can be performed by the image processing device 10 may include an image acquisition step S31, a difference map generation step S34, a motion map generation step S35, a cumulative motion map generation step S36, a statistical value map (for example, entropy map) generation step S38, and an image output step S37. The image processing method produces a noise-reduced output image through a recursive way. In this regard, this method may also be referred to as an infinite recursive depth.

The image input unit 11 receives an input image from an external source. For example, the image input unit 11 may sequentially receive each frame of video captured by the previously mentioned image acquisition unit 120 over time. An image composed of multiple pixels of a region including the subject can be captured over time as a radiation video sequence by the image acquisition unit 120, which is the radiation detection panel, and the acquired radiation video sequence can be input to the image input unit 11 in chronological order.

The input image may be one frame of a video consisting of multiple frames. Also, the input image can be a two-dimensional image with multiple pixels including a plurality of rows (n rows) and a plurality of columns (m columns) and may include noise along with the captured subject. For example, noise included in the input image may include a line noise in a predetermined direction, for instance, in a horizontal and/or vertical direction, and/or random noise.

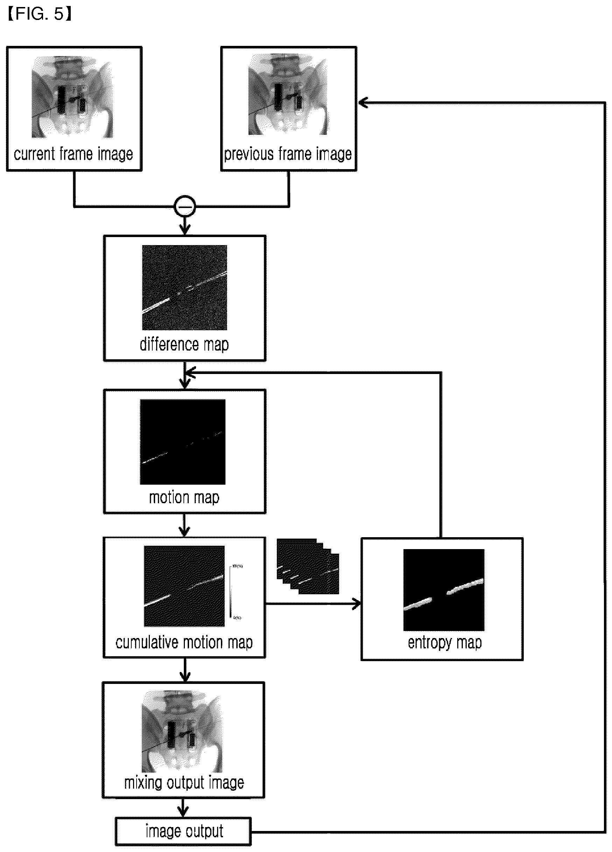

The motion map is generated through the generation of a difference map by the difference unit 14 and a threshold processing of the difference map by the threshold processing unit 15. As a result, as shown in FIG. 5, a motion map can be generated from the noise-processed current frame image and the previous frame image, for example, the output image of the previous frame. The motion map is obtained from the data derived by subtracting the previous frame image from the current frame image and includes information about the motion status of each pixel. For instance, pixels with motion can be assigned a value of '0', and pixels without motion can be assigned a value of '1'. Thus, all pixels in the motion map have a pixel value of either 0 or 1. Pixels with a value of 0 indicate motion relative to the previous frame image, and pixels with a value of 1 indicate no motion relative to the previous frame image. The determination of motion can be based on threshold processing, and in this embodiment of the invention, a statistical value map, such as an entropy map, is used for motion determination.

The difference unit 14 creates a difference map by subtracting, for example, the output image of the previous frame from the current frame image. Before this subtraction, noise processing, such as noise reduction and noise stabilization, can be performed on both the current frame image and the output image of the previous frame. By using the output image of the previous frame in the generation of the difference map for the current frame, noise reduction processing in a recursive structure can be achieved. The difference unit 14 can create a difference map by calculating the pixel value difference of each pixel at the same position between the current frame image and the previous frame image. In other words, the difference map contains the same number of pixels as the frame image, and the value of each pixel in the difference map corresponds to the difference value of the respective pixel between the current frame image and the previous frame image. It is noted that the term "previous frame image" here refers not only to the image of the frame immediately preceding the current frame but also can encompass images from frames even before that. In this context, the difference map can be derived by subtracting one or more frame images, for example, output images, from the current frame's image, which might be an input image. For instance, the difference map may be generated by subtracting any one output image among the previous frames from the current frame's input image. Alternatively, the difference map could be derived by subtracting a blended image, obtained by mixing output images of a plurality of previous frames, from the current frame's input image.

The difference map can contain motion information of the subject and residual noise information. Additionally, the generated difference map can be stabilized using filters such as a mean filter or a median filter.

The threshold processing unit 15 determines a threshold for motion detection in the difference map and generates a motion map based on each pixel's motion status by performing threshold processing based on the determined threshold. In other words, the threshold processing unit 15 discriminates the motion status of each pixel in the difference map through threshold processing and generates a motion map consisting of information indicating the motion status of each pixel. If the threshold for motion detection is set too low, the sensitivity to motion detection increases, reducing the level of noise reduction. In contrast, if the threshold is set too high, the sensitivity to motion detection decreases, which can result in motion blur (dragging effect). Since X-ray images are acquired under various radiation conditions and subject characteristics, it is difficult to predict the pixel values of the acquired image, and it is necessary to set an appropriate threshold based on pixel values.

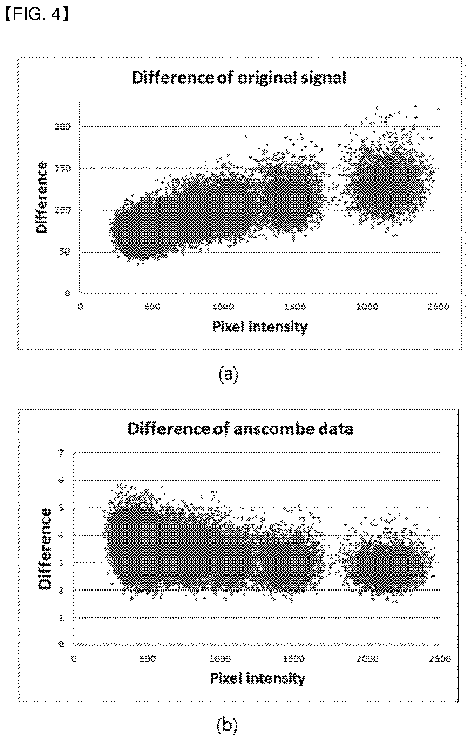

The threshold for motion detection can be determined through various methods such as statistical methods, experimental methods, and arithmetic methods. FIG. 4 comparatively shows the distribution of pixel values from the difference map of the original signal for the same input image (previous frame and current frame) of a stationary subject, and the distribution of pixel values from the difference map obtained after the Anscombe transform. FIG. 4 (a) shows a graph depicting the pixel value distribution of the difference map derived from the original signal, while FIG. 4 (b) shows a graph illustrating the pixel value distribution of the difference map obtained after the Anscombe transform of the original signal. In the graphs of FIG. 4, the horizontal axis (x-axis) represents the pixel value (intensity), and the vertical axis (y-axis) indicates the difference in pixel values at the same location between the previous frame and the current frame. Since the subject is stationary, the distribution of data in the y-axis direction for any arbitrary x value may be considered as noise, and pixels where the distribution of data in the y-axis direction exceeds the noise value can be estimated as pixels where motion has occurred.

For instance, in the distribution of the difference map of the original signal, if the pixel value is 500, a suitable threshold for motion detection is approximately 100, and if the pixel value is 2200, a suitable threshold is about 170. Due to such significant differences in the appropriate threshold depending on pixel values, deciding on a threshold becomes challenging. In contrast, in the distribution of the difference map of the signal transformed by the Anscombe transform as shown in FIG. 4 (b), if the pixel value is 500, a suitable threshold is about 5, and if the pixel value is 2200, a threshold of about 4 is suitable. Thus, when generating a difference map by differentiating the Anscombe transformed signal, the difference in the appropriate threshold depending on pixel values diminishes significantly, making threshold determination much easier. In this regard, the threshold can be set, for instance, to a value that falls within the top 70 to 80% range of the distribution of pixel value differences for a specific pixel value.

Furthermore, even when deriving difference data from the Anscombe transformed signal, there is still a slight deviation when applying the same threshold to all pixel values. Taking this into account, adaptive thresholding can be used to evenly adjust the noise deviation across all pixel values. The reason the difference value changes non-linearly depending on the pixel value is due to the mathematical characteristics of the Anscombe transform.

The threshold processing unit 15 performs threshold processing on the difference map generated by the difference unit 14 to generate a motion map. The threshold processing unit 15 applies an adaptive threshold based on the statistical value map (for example, the entropy map) generated by the statistical value map generation unit 18 which will be described later, to produce a motion map from the difference map. The method of determining motion detection using the adaptive threshold and generating a motion map accordingly will be explained below.

The threshold processing unit 15 determines whether each pixel in the difference map has motion by comparing its pixel value to a predetermined threshold. If the pixel value is greater than or equal to the threshold, it is determined the pixel with motion and the pixel is assigned a value indicating motion, e.g. '0'. In contrast, if the pixel value is less than the threshold, it is determined a pixel without motion and the pixel is assigned a value indicating no motion, e.g. '1'. The threshold processing unit 15 performs this motion determination for all pixels in the difference map using the threshold and assigns the resulting values to the respective pixels, thereby generating a motion map. In this way, the threshold processing unit 15 generates a motion map using the difference map and an adaptive threshold curve indicating the adaptive threshold. Consequently, the motion map has the same pixels as the input frame image and contains a value indicating the presence or absence of motion for each pixel.

In another embodiment of the invention, threshold processing may not occur on a per-pixel basis but can be performed on a mask unit that includes a plurality of adjacent pixels. For example, a mask can be set up to include 9 pixels in a 3x3 arrangement surrounding a central pixel. The motion status of the central pixel can be determined by comparing the average or median value of the pixels within that mask to the threshold. By moving the mask in this manner, motion determination can be carried out on a mask-by-mask basis, leading to the generation of a motion map. By performing threshold processing on a mask unit, noise present in a specific pixel can be reduced.

As explained above, the threshold processing unit 15 determines the motion status for each pixel, assigning different values to pixels determined to have motion and pixels determined not to have motion, and consequently every pixel in the difference map will take one of two predetermined values, e.g. either 0 or 1. The map obtained in this manner contains information about the motion status of all pixels, and in this sense, this map can be referred to as a motion map.

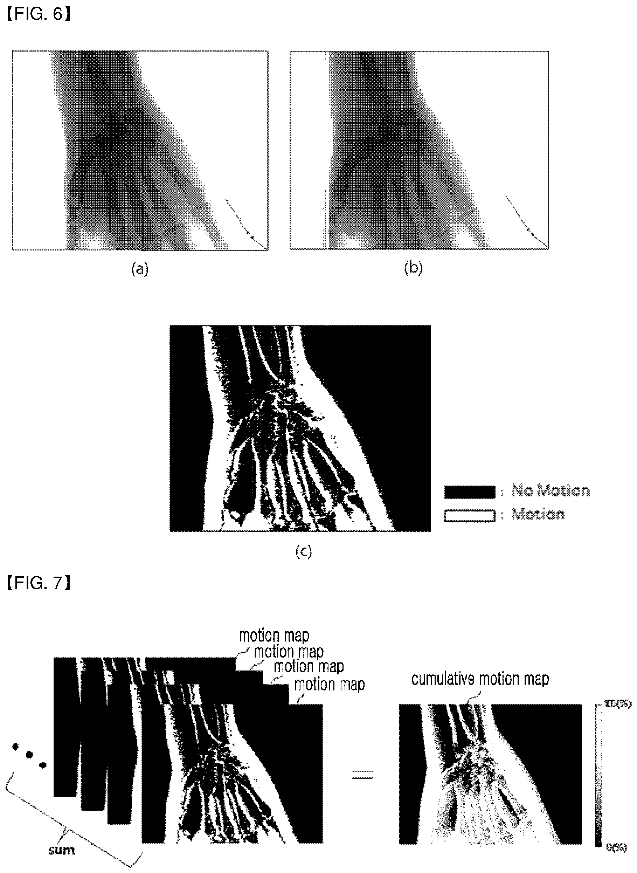

FIG. 6 shows an example of a motion map output when the hand phantom is moved from the right to the left in the previous frame image and the current frame image. FIG. 6 (a) shows the previous frame image including the hand phantom, and FIG. 6 (b) shows the current frame image in which the hand phantom moved from the right to the left. FIG. 6 (c) shows the output motion map displaying the motion detection result for each pixel of the difference data.

Referring to FIG. 6 (c), the areas marked in white indicate pixels where motion is detected. Within the hand phantom where actual motion is present, areas with high contrast to the previous frame (edges of the bone region) are detected as motion, while areas with low contrast to the previous frame (skin areas, tool regions) are not detected as motion. Consequently, in subsequent processes when generating the final output image, areas with motion can be updated based primarily on the current frame, while the remaining areas can be averaged. This reduces noise and strengthens edges, and thereby a sharpness is enhanced and a motion blur can be prevented.

The cumulative motion map generation unit 16 accumulates motion maps based on motion detection results in a separate memory over time and sums them to generate a cumulative motion map. The cumulative motion map is used to determine the appropriate mixing ratio of the previous frame image and the current frame image to generate the final output image.

FIG. 7 illustrates a method of generating a cumulative motion map using the motion map. The cumulative motion map generation unit 16 accumulates the motion map obtained through the aforementioned motion detection in a separate memory for each frame in chronological order to generate and update the cumulative motion map. The image on the left of FIG. 7 represents the cumulative motion map, while the right shows the cumulative motion map resulting from the summation of motion maps. The cumulative motion map of the current frame can be generated by summing the values of the same pixels from the cumulative motion map of the previous frame and the motion map of the current frame. In other words, the cumulative motion map of a particular frame becomes the image obtained by summing the pixel values of the same pixels from all motion maps obtained up to that frame. For instance, referring to FIG. 7, if the current frame is the 4th frame, the sum of the values from the same pixel in the motion maps obtained from the 4 frames so far becomes the value of the same pixel in the cumulative motion map of the current frame.

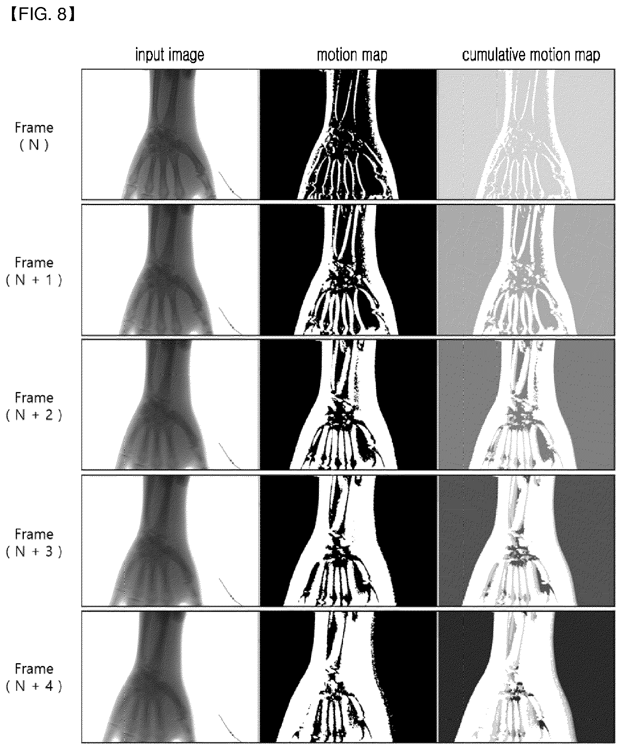

In the motion map, if pixels determined to have motion are assigned a value of '0', making them bright, and pixels determined to not have motion are assigned a value of '1', making them dark, then each pixel in the cumulative motion map will have a value corresponding to the sum of that pixel's motion detection values (either 0 or 1). This results in different values or brightness levels depending on the number of motion detections. For instance, considering a cumulative motion map composed of 10 frames, this cumulative map is derived from the summation of the 10 motion maps, and each pixel in the cumulative motion map can have any value ranging from 0 to 10. If all the values of the same pixel in every motion map are 0, then the corresponding pixel in the cumulative motion map will have a value of 0. In contrast, if all the values of the same pixel in every motion map are 1, then the corresponding pixel in the cumulative motion map will have a value of 10. Consequently, as depicted in FIG. 8, each pixel in the cumulative motion map will have a brightness value determined by the number of motion detections of the same pixel in all the motion maps up to that frame.

In this context, in the generated cumulative motion map, the brighter the pixel value, the higher the probability of motion, and the darker the pixel value, the lower the probability of motion. For instance, at the same pixel location, as frames progress over time, if motion is detected, the probability of motion increases, whereas if motion is not detected, the probability of motion decreases.

Each pixel value in the cumulative motion map represents the degree of motion or the probability value of the motion at that pixel. That is, if in each frame's motion map, a value of '0' is assigned when there is motion and '1' when there is no motion, then a smaller pixel value in the cumulative motion map indicates a higher probability of motion at that pixel.

In another embodiment of the invention, instead of accumulating the pixel values of the motion map when updating the cumulative motion map, the cumulative motion map can be configured to selectively increase or decrease the pixel values of the cumulative motion map using a threshold-processed statistical value map.

FIG. 8 shows an example of the process of generating motion maps and cumulative motion maps based on the input image as the frame progresses. The motion map of each frame can be generated from the difference between the frame images of the current frame and the previous frame, and the cumulative motion map of the current frame can be generated from the summation of the generated motion map and the cumulative motion map of the previous frame.

Meanwhile, according to an embodiment of the invention, the entropy map generation unit 18 generates a statistical value map for adaptive threshold processing used to generate the motion map. The statistical value map is generated from the cumulative motion map and is formed to have the same number of pixels as the cumulative motion map. Each pixel of the cumulative value map has a pixel value that represents a statistical value indicating the probability of motion occurrence at that pixel in the cumulative motion map. For example, a mask that includes multiple pixels, including the specific pixel in question, can be set in the cumulative motion map, and a statistical value indicating the probability of motion occurrence in the mask can be set as the statistical value of the specific pixel. The statistical value representing the probability of motion at a specific pixel can be an entropy value, a standard deviation, etc. of the pixel values of a plurality of pixels surrounding the specific pixel. Hereinafter, the case where an entropy value is used as a statistical value is exemplarily described, and in this sense, the statistical value map is referred to as an entropy map. The entropy map is created from the cumulative motion map using the entropy concept, a statistical approach. The generated entropy map can be stored in a separate buffer memory and can be used to generate a motion map of the next frame. The motion map of the current frame can be generated using the entropy map generated in the previous frame, and if necessary, the motion map of the current frame may be regenerated using the entropy map generated in the current frame. At this time, the motion map of the current frame can be generated using not only the entropy map of the immediately preceding frame but also one or more entropy maps of previous frames.

Since the motion map is generated based on the difference map obtained from the difference between the current frame image and the previous frame image, in some cases, it may not accurately detect the motion of the subject. For instance, if the subject is thin, the difference values, i.e., the pixel values of each pixel in the difference map, might not be sufficiently above the noise level, making it difficult to detect motion. For example, it might be difficult to detect the motion of a thin and sharp object, like a needle, when it passes through a thick part of a patient. To address this issue, in embodiments of the present invention, the motion map is generated by applying an adaptive thresholding, which applies a different threshold value for each pixel depending on the conditions.

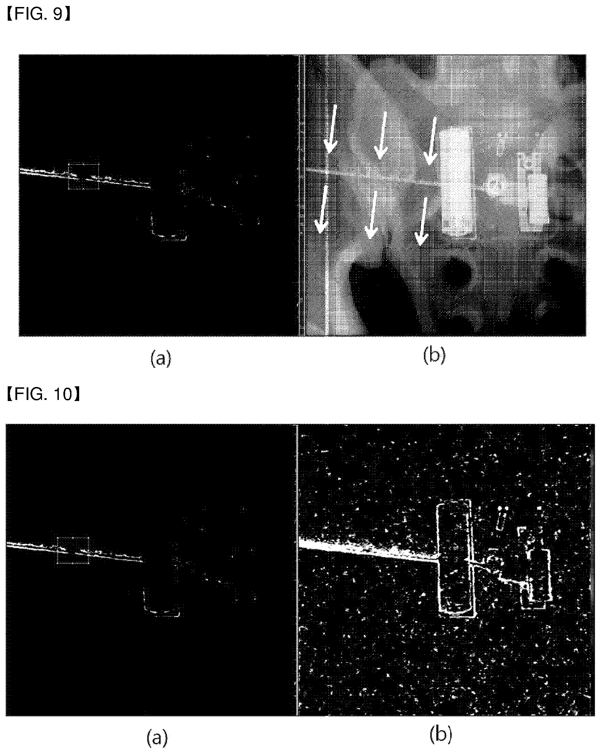

FIG. 9 shows a motion map and an output image obtained when a thin lead bar passes over a stationary pelvic phantom, and these images were obtained without the application of the adaptive thresholding according to an embodiment of the present invention. FIG. 9 (a) shows a motion map, while FIG. 9 (b) shows an output image. The arrows indicated in FIG. 9 (b) represent the direction of motion. Referring to FIG. 9, in the area where the bar passes over the thinner parts of the phantom, the difference in values before and after the bar's passage is significant enough for the pixels to be accurately detected as in motion. However, when the bar moves over the thicker bone regions, the difference in values is not sufficient, causing those pixels to not be detected as moving. In this case, these pixels are considered as noise and get averaged during the generation of the output image, resulting in the bar appearing as if it's broken in the final output image.

FIG. 10 shows the motion map obtained under the same conditions as FIG. 9. FIG. 10 (a) shows a motion map when the threshold for motion detection is increased, and FIG. 10 (b) shows the motion map when the threshold is decreased. As illustrated in FIG. 10 (b), if the threshold for motion detection is reduced to prevent the bar from breaking, noise may be considered as moving pixels, leading to an unintended side effect where the noise is amplified. Thus, there exists a trade-off depending on the threshold when detecting the motion of thin objects.

An embodiment of the present invention employs the concept of entropy, a statistical approach, to address these issues. Entropy quantitatively represents the certainty or amount of information in a probability distribution. If the probability of a specific value in a probability distribution increases and the probabilities of other values decrease, the entropy diminishes. Conversely, if various values have similar probabilities, the entropy increases. Entropy may be regarded as one of the characteristic values that represents the distribution characteristics of a probability distribution. If the probability or probability density is concentrated around a specific value, the entropy can be considered low, and if the probability or probability density spreads evenly across various values, the entropy can be considered high. That is, the higher the entropy, the more uneven and information-rich it is; the lower the entropy, the more uniform and less informative it is.

As known, in statistics, entropy H represents an average amount of information, and for a discrete random variable X, it can be defined as shown in Equation 1.

[Equation 1]

$$H(x) = E[I(X)] = - \sum_{i=1}^{n} P(x_i) \log_b (P(x_i))$$

Here, n represents the number of frequency items, i.e., the number of motion probability values that X can have, and P represents for the frequency of each frequency item, i.e., the probability mass function indicating the frequency of each motion probability value.

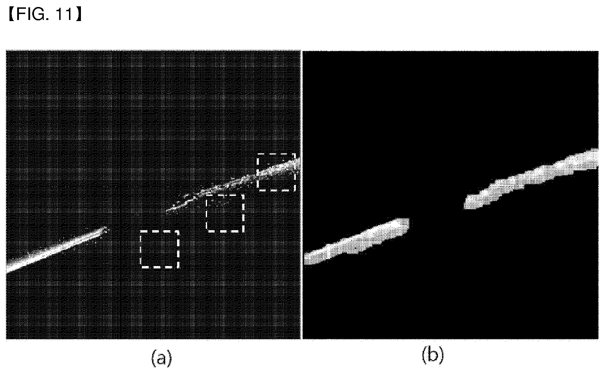

The entropy of motion probability values can be calculated for each pixel of the cumulative motion map, or for a set of a plurality of pixels, and by assigning the calculated entropy to each pixel or the set of a plurality of pixels, an entropy map can be generated. For example, a masking area is selected for respective pixels in the cumulative motion map, and an entropy value of the motion probabilities of pixels within the corresponding masking area is calculated, and then the calculated entropy value is assigned to the pixel value of the corresponding pixel to generate an entropy map. A sufficiently large area, including the pixel of interest, can be selected as a mask, and the entropy of motion probabilities within this mask can be calculated using a histogram. In this regard, the histogram can be derived with motion probability values as frequency items and the occurrence counts of each motion probability value as the frequency, and the entropy can then be calculated using the aforementioned Equation 1. For instance, if the cumulative motion map is an image of 1500x1500 pixels, a 300x300 mask including the target center pixel can be set, and the entropy within this mask can be calculated and set as the entropy for the center pixel. FIG. 11 illustratively shows an entropy map generated by computing entropy through pixel masking calculations in a cumulative motion map. FIG. 11 (a) shows an example of a cumulative motion map, where masks are denoted by dashed lines. Entropy is calculated for each mask, and among the three masks depicted in FIG. 11 (a), the rightmost mask, which contains the brightest pixels (indicating high motion probability), can be predicted to have the highest entropy. Conversely, the leftmost mask, which contains the darkest pixels (indicating low motion probability), is expected to have the lowest entropy. FIG. 11 (b) shows an entropy map generated by computing the entropy for all pixels in the cumulative motion map of 11 (a) within the defined masks. Accordingly, each pixel in the entropy map has the entropy value of the motion probability values of pixels contained in the mask that includes the given pixel, as a pixel value. Pixels or areas with high entropy may be considered to have a high probability of motion, while those with low entropy likely have a low probability of motion. In this context, entropy may be regarded as a measure representing the complexity of motion probability values.

In an embodiment of this invention, the generated entropy map is used to generate the motion map, and the entropy of each pixel contained in the entropy map is utilized to adjust the threshold for motion detection. The generated motion map is stored in a separate buffer memory, and adaptive threshold processing based on this entropy map, i.e., the entropy map generated from the previous frame, is carried out when generating the motion map for the next frame. The threshold for each pixel used in the thresholding process performed by the thresholding unit can be set in inverse proportion to the size of the entropy of the corresponding pixel in the entropy map. For example, during motion map generation, since pixels or regions with high entropy may also have a high probability of having moved previously, their motion detection thresholds are lowered, allowing for more sensitive motion detection. Conversely, since pixels or regions with low entropy may also have a lower probability of having moved previously, their motion detection thresholds are raised, allowing for a more conservative detection approach. This adaptive thresholding process can help improve the trade-off issue related to the magnitude of the threshold mentioned earlier. Meanwhile, when variance or standard deviation is used as the statistical value instead of entropy, pixels or regions with small variance or standard deviation may be considered to have a higher probability of having moved previously.

By generating a motion map through the adaptive thresholding based on entropy, not only is there an improvement in motion detection accuracy for individual frames, but even if accurate motion detection is not achieved in every frame, the amount of information accumulates as the frames progress and this has the effect of gradually expanding the region by lowering the threshold for pixels around moving objects, and as a result, as the frames progress, the motion detection accuracy for slim objects significantly improves.

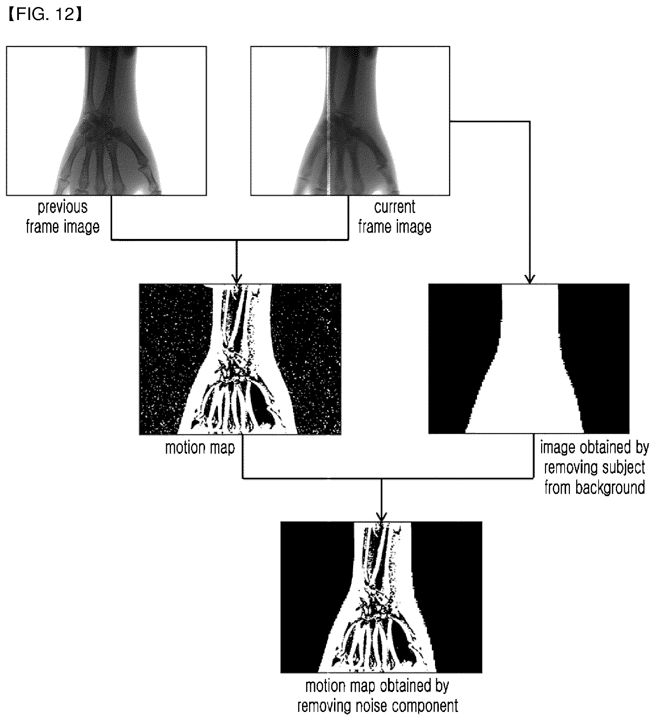

FIG. 12 shows a method of generating a motion map according to another embodiment of the present invention. Despite generating a motion map through the threshold processing based on the noise level of the image in the previously described embodiment, there may still be noise components left in the motion map. Therefore, in this embodiment, additional noise reduction processing is performed.

In this embodiment, remaining noise is removed by separating the subject from the background and excluding the background area from the motion map. A background image, from which the subject has been removed, is generated by experimentally applying a threshold to the current frame or through artificial intelligence training, and then, by performing an addition or multiplication operation between the motion map generated by the previous embodiment and the generated background image, and thereby a motion map with noise components removed is produced. As a result, noise components remaining in the background can be eliminated, and a motion map containing only information about the subject's motion can be generated. This method provides an advantage of being able to sensitively detect the motion of the subject while the overall noise level of the image increases slightly when the motion detection threshold is lowered.

In another embodiment of the present invention, when setting a threshold for motion detection, the background and the subject area can be distinguished and different thresholds are applied thereto, thereby creating two separate motion maps. Subsequently, the two generated motion maps can be merged to produce a final motion map.

The output image generation unit 17 combines the current frame image with the previous frame image to produce an output image. In this regard, the output image generation unit 17 combines the current frame image and the output image of the previous frame with an appropriate mix ratio based on the cumulative motion map to generate the final output image. As the value of each pixel in the cumulative motion map represents a greater extent of motion, the proportion reflecting the current frame image can be set to increase relatively. That is, the mix ratio can be determined such that the weight of the current frame increases as the motion probability determined by the pixel value contained in the cumulative motion map increases. For example, on a pixel-by-pixel basis, the ratio of reflecting the current frame image increases linearly as the pixel value of the cumulative motion map indicating the extent of motion increases.

As a concrete example, for pixels with more motions, the corresponding pixel of the current frame image is given a predetermined weight α1, for instance, 0.8, and the corresponding pixel of the cumulative output image till the previous frame is given a weight 1-α1, for example, 0.2. By performing this mixing process for all pixels, the current frame image and the output image of the previous frame can be mixed. On the other hand, for pixels with less motion, the corresponding pixel of the current frame image is given a predetermined weight α2, for instance, 0.2, and the respective pixel of the cumulative previous frame image is given a weight 1-α2, for example, 0.8, allowing the current frame image and the previous frame output image to be mixed. When the weight of the current frame is higher for pixels with more motions, a motion-blur-free image can be obtained. As a result, the final output image is updated by reflecting more of the current frame's values for pixels with more motions and reflecting more of the previous frame's values for pixels with less motion, and accordingly as frames accumulate over time, noise reduction performance can be improved.

Ultimately, after outputting the noise-reduced image, the result is stored in a memory. When generating the output image for the next frame, this stored image is used as the previous frame's image, implementing a recursive way.

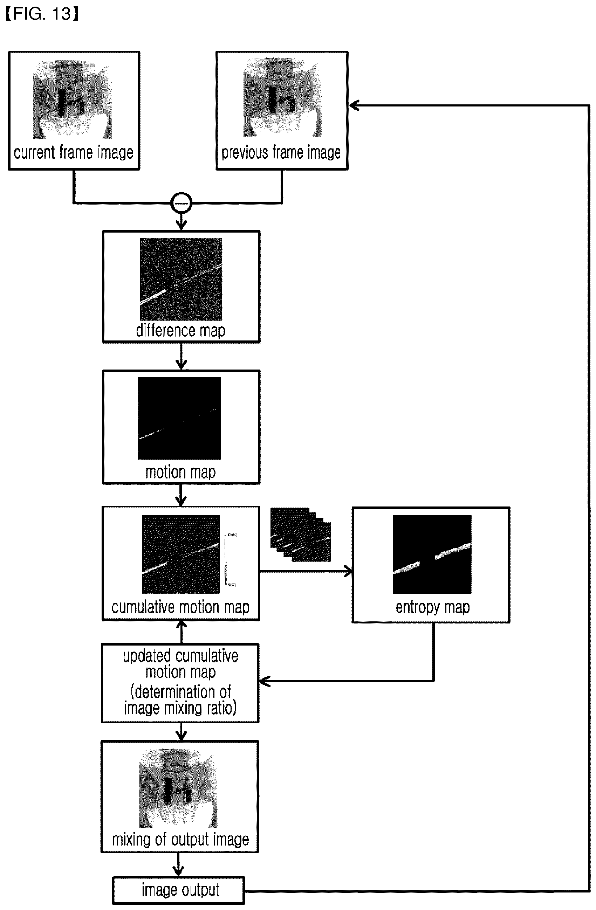

FIG. 13 shows the process of an image processing method according to another embodiment of the present invention. As shown in FIG. 13, the entropy map generated from the cumulative motion map is used when generating the output image through the mixture of the current frame image and the previous frame image. As described in the previous embodiment, the mixing ratio of the current frame image and the previous frame image is determined by the cumulative motion map, and a pixel with a high entropy value represents a high probability of motion occurring in the previous frame.

Thresholding of the entropy map can determine pixels that are determined to have motion in the entropy map. The threshold for this thresholding process can be determined experimentally in advance or can be set while viewing the real-time image quality through a user interface as a sensitivity. For instance, if the mask size for entropy calculation during the generation of the entropy map is determined, the maximum possible value of entropy can be set. A threshold can be set to the value below 20% of this maximum entropy value.

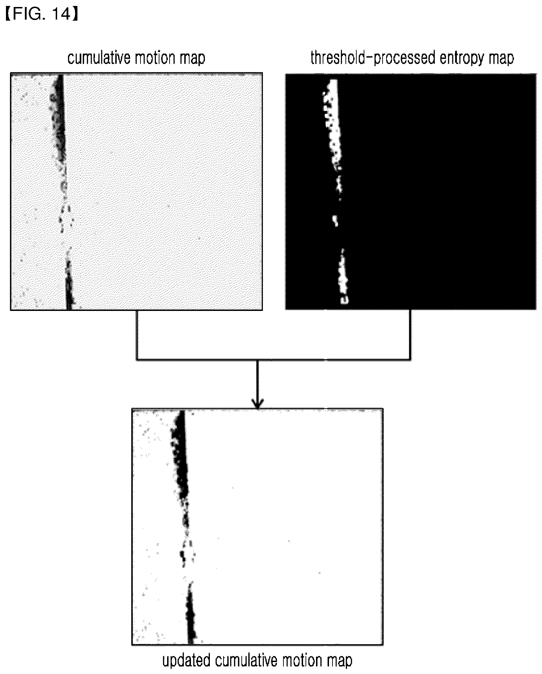

In this embodiment, thresholding is performed on the entropy map, and as illustratively depicted in FIG. 14, the cumulative motion map is updated using the thresholded entropy map.

The invention is defined by the appended claims.

[Industrial Applicability]

The present invention relates to a method for processing images obtained by radiation imaging and can be applied to image processing technology, so it has an industrial applicability.

**Claims**

1. 1. An image processing device for performing noise reduction processing on images obtained by radiation imaging using a recursive way, comprising:

an image input unit (11) configured to receive frame-by-frame images obtained by the radiation imaging;
a motion map generation unit configured to generate a motion map having motion detection information for each pixel of a difference map obtained by a difference between a current frame image and a previous frame image, the motion map generation unit comprising: a difference map generation unit (14) configured to generate the difference map based on the difference between the current frame image and the previous frame image; and a threshold processing unit (15) configured to generate the motion map having the motion detection information for each pixel through a threshold processing of pixel values of the difference map;
a cumulative motion map generation unit (16) configured to generate a cumulative motion map based on the generated motion map and a cumulative motion map up to a previous frame;
a statistical value map generation unit (18) configured to generate a statistical value map formed by assigning a statistical value representing a motion probability of each pixel of the cumulative motion map; and
an output image generation unit (17) that generates an output image of a current frame by mixing the current frame image and the output image of the previous frame based on the cumulative motion map;

**characterized in that**

the threshold processing unit (15) is configured to set a threshold value of each pixel for the threshold processing lower as a motion probability indicated by the statistical value of a pixel corresponding to the statistical value map generated in the previous frame is higher;

the statistical value map generation unit (18) is configured to calculate the statistical value based on a pixel mask that comprises a plurality of pixels comprising each pixel of the cumulative motion map and assigns the calculated statistical value to each pixel to generate the statistical value map, the statistical value being an entropy, a variance, or a standard deviation of the pixel values of the plurality of pixels in the pixel mask.

2. The imaging processing device of claim 1, wherein the difference map is obtained using an image of the current frame and one or more images of the previous frame,

wherein the previous frame image is an output image of the previous frame, or

wherein the cumulative motion map generation unit (16) is configured to update the cumulative motion map using the statistical value map,

wherein the output image generation unit (17) is preferably configured to determine a mixing ratio of the current frame image and the output image of the previous frame based on the updated cumulative motion map.

3. A computer-implemented image processing method for processing an image obtained by radiation imaging for reducing noise through a recursive way, comprising:

receiving (S31) frame-by-frame images obtained through the radiation imaging;

generating (S34, S35) a motion map that includes motion detection information for each pixel of a difference map obtained by a difference between a current frame image and a previous frame image, wherein in the generating of the motion map, the motion map having motion detection information for each pixel is generated by a thresholding for pixel values of each pixel of the difference map;

generating (S36) a cumulative motion map based on the generated motion map and the motion map accumulated up to a previous frame;

generating (S38) a statistical value map formed by assigning statistical values representing a motion probability of a plurality of pixels including each pixel of the cumulative motion map; and

mixing (S37) the current frame image and an output image of the previous frame based on the cumulative motion map to produce an output image of the current frame,

**characterized in that**

a threshold of each pixel for the thresholding is set lower as a motion probability indicated by the statistical value of the corresponding pixel of the statistical value map

generated in the previous frame increases,

in the generating of the statistical value map, the statistical value map is generated by calculating the statistical value based on a pixel mask which comprises a plurality of pixels having each pixel of the cumulative motion map and then assigning the calculated statistical value to each pixel, wherein the statistical value is an entropy, a variance, or a standard deviation of pixel values of the plurality of pixels of the pixel mask.

4. The image processing method of claim 3, wherein the difference map is obtained using an image of the current frame and one or more images of the previous frames.

5. The image processing method of claim 3, further comprising updating the cumulative motion map using the statistical value map,

wherein in the generating of the output image, a mixing ratio of the current frame image and the output image of the previous frame is preferably determined based on the updated cumulative motion map.

**Patentansprüche**

1. Bildverarbeitungsvorrichtung zum Durchführen einer Rauschreduktionsverarbeitung an Bildern, die durch Strahlungsbildgebung erhalten werden, unter Verwendung einer rekursiven Weise, umfassend:

eine Bildeingabeeinheit (11), die konfiguriert ist, um Einzelbild-für-Einzelbild-Bilder zu empfangen, die durch die Strahlungsbildgebung erhalten werden;

eine Bewegungskartenerzeugungseinheit, die konfiguriert ist, um eine Bewegungskarte mit Bewegungsdetektionsinformationen für jedes Pixel einer Differenzkarte zu erzeugen, die durch eine Differenz zwischen einem aktuellen Einzelbild und einem vorherigen Einzelbild erhalten wird, wobei die Bewegungskartenerzeugungseinheit umfasst: eine Differenzkartenerzeugungseinheit (14), die konfiguriert ist, um die Differenzkarte basierend auf der Differenz zwischen dem aktuellen Einzelbild und dem vorherigen Einzelbild zu erzeugen; und eine Schwellenwertverarbeitungseinheit (15), die konfiguriert ist, um die Bewegungskarte mit den Bewegungsdetektionsinformationen für jedes Pixel durch eine Schwellenwertverarbeitung von Pixelwerten der Differenzkarte zu erzeugen;

eine Erzeugungseinheit (16) für eine kumulative Bewegungskarte, die konfiguriert ist, um eine kumulative Bewegungskarte basierend auf der erzeugten Bewegungskarte und einer kumulativen Bewegungskarte bis zu einem vorherigen Einzelbild zu erzeugen;

eine Statistikwertkartenerzeugungseinheit (18), die konfiguriert ist, um eine Statistikwertkarte zu erzeugen, die durch Zuweisen eines Statistikwerts gebildet wird, der eine Bewegungswahrscheinlichkeit jedes Pixels der kumulativen Bewegungskarte darstellt; und

eine Ausgabebilderzeugungseinheit (17), die ein Ausgabebild eines aktuellen Einzelbilds durch Mischen des aktuellen Einzelbilds und des Ausgabebilds des vorherigen Einzelbilds basierend auf der kumulativen Bewegungskarte erzeugt;

**dadurch gekennzeichnet, dass**

die Schwellenwertverarbeitungseinheit (15) konfiguriert ist, um einen Schwellenwert jedes Pixels für die Schwellenwertverarbeitung niedriger einzustellen, wenn eine Bewegungswahrscheinlichkeit, die durch den Statistikwert eines Pixels angezeigt wird, das der im vorherigen Einzelbild erzeugten Statistikwertkarte entspricht, höher ist;

die Statistikwertkartenerzeugungseinheit (18) konfiguriert ist, um den Statistikwert basierend auf einer Pixelmaske zu berechnen, die eine Vielzahl von Pixeln umfasst, die jedes Pixel der kumulativen Bewegungskarte umfassen, und den berechneten Statistikwert jedem Pixel zuweist, um die Statistikwertkarte zu erzeugen, wobei der Statistikwert eine Entropie, eine Varianz oder eine Standardabweichung der Pixelwerte der Vielzahl von Pixeln in der Pixelmaske ist.

2. Bildverarbeitungsvorrichtung nach Anspruch 1, wobei die Differenzkarte unter Verwendung eines Bilds des aktuellen Einzelbilds und eines oder mehrerer Bilder des vorherigen Einzelbilds erhalten wird,

wobei das vorherige Einzelbild ein Ausgabebild des vorherigen Einzelbilds ist, oder wobei die kumulative Bewegungskartenerzeugungseinheit (16) konfiguriert ist, um die kumulative Bewegungskarte unter Verwendung der Statistikwertkarte zu aktualisieren, wobei die Ausgabebilderzeugungseinheit (17) vorzugsweise konfiguriert ist, um ein Mischverhältnis des aktuellen Einzelbilds und des Ausgabebilds des vorherigen Einzelbilds basierend auf der aktualisierten kumulativen Bewegungskarte zu bestimmen.

3. Computerimplementiertes Bildverarbeitungsverfahren zum Verarbeiten eines durch Strahlungsbildgebung erhaltenen Bilds zum Reduzieren von Rauschen durch eine rekursive Weise, umfassend:

Empfangen (S31) von Einzelbild-für-Einzelbild-Bildern, die durch die Strahlungsbildgebung erhalten werden;

Erzeugen (S34, S35) einer Bewegungskarte, die Bewegungsdetektionsinformationen für jedes Pixel einer Differenzkarte enthält, die durch eine Differenz zwischen einem aktuellen Einzelbild und einem vorherigen Einzelbild erhalten wird, wobei beim Erzeugen der Bewegungskarte die Bewegungskarte mit Bewegungsdetektionsinformationen für jedes Pixel durch eine Schwellenwertbildung für Pixelwerte jedes Pixels der Differenzkarte erzeugt wird;

Erzeugen (S36) einer kumulativen Bewegungskarte basierend auf der erzeugten Bewegungskarte und der Bewegungskarte, die bis zu einem vorherigen Einzelbild akkumuliert wurde;

Erzeugen (S38) einer Statistikwertkarte, die durch Zuweisen von Statistikwerten gebildet wird, die eine Bewegungswahrscheinlichkeit einer Vielzahl von Pixeln darstellen, die jedes Pixel der kumulativen Bewegungskarte enthalten; und

Mischen (S37) des aktuellen Einzelbilds und eines Ausgabebilds des vorherigen Einzelbilds basierend auf der kumulativen Bewegungskarte, um ein Ausgabebild des aktuellen Einzelbilds zu erzeugen,

**dadurch gekennzeichnet, dass**

ein Schwellenwert jedes Pixels für die Schwellenwertbildung niedriger eingestellt wird, wenn eine Bewegungswahrscheinlichkeit, die durch den Statistikwert des entsprechenden Pixels der Statistikwertkarte, die in dem vorherigen Einzelbild erzeugt wird, angegeben wird, zunimmt,

beim Erzeugen der Statistikwertkarte die Statistikwertkarte durch Berechnen des Statistikwerts basierend auf einer Pixelmaske erzeugt wird, die eine Vielzahl von Pixeln umfasst, die jedes Pixel der kumulativen Bewegungskarte aufweisen, und dann Zuweisen des berechneten Statistikwerts zu jedem Pixel,

wobei der Statistikwert eine Entropie, eine Varianz oder eine Standardabweichung von Pixelwerten der Vielzahl von Pixeln der Pixelmaske ist.

**4.** Bildverarbeitungsverfahren nach Anspruch 3, wobei die Differenzkarte unter Verwendung eines Bilds des aktuellen Einzelbilds und eines oder mehrerer Bilder der vorherigen Einzelbilder erhalten wird.

**5.** Bildverarbeitungsverfahren nach Anspruch 3, ferner umfassend Aktualisieren der kumulativen Bewegungskarte unter Verwendung der Statistikwertkarte,
wobei beim Erzeugen des Ausgabebilds ein Mischverhältnis des aktuellen Einzelbilds und des Ausgabebilds des vorherigen Einzelbilds vorzugsweise basierend auf der aktualisierten kumulativen Bewegungskarte bestimmt wird.

**Revendications**

**1.** Dispositif de traitement d'images destiné à effectuer un traitement de réduction de bruit sur des images obtenues par imagerie par rayonnement à l'aide d'une méthode récursive, comprenant :

une unité d'entrée d'images (11) configurée pour recevoir des images trame par trame obtenues par l'imagerie par rayonnement ;
une unité de génération de carte de mouvement configurée pour générer une carte de mouvement comportant des informations de détection de mouvement pour chaque pixel d'une carte de différence obtenue par une différence entre une image de trame actuelle et une image de trame précédente, l'unité de génération de carte de mouvement comprenant : une unité de génération de carte de différence (14) configurée pour générer la carte de différence sur la base de la différence entre l'image de trame actuelle et l'image de trame précédente ; et une unité de traitement de seuil (15) configurée pour générer la carte de mouvement comportant les informations de détection de mouvement pour chaque pixel par un traitement de seuil des valeurs de pixel de la carte de différence ;
une unité de génération de carte de mouvement cumulative (16) configurée pour générer une carte de mouvement cumulative sur la base de la carte de mouvement générée et d'une carte de mouvement cumulative jusqu'à une trame précédente ;
une unité de génération de carte de valeurs statistiques (18) configurée pour générer une carte de valeurs statistiques formée en attribuant une valeur statistique représentant une probabilité de mouvement de chaque pixel de la carte de mouvement cumulative ; et
une unité de génération d'image de sortie (17) qui génère une image de sortie d'une trame actuelle en mélangeant l'image de trame actuelle et l'image de sortie de la trame précédente sur la base de la carte de mouvement cumulative ;
**caractérisé en ce que**
l'unité de traitement de seuil (15) est configurée pour définir une valeur de seuil de chaque pixel pour le traitement de seuil plus faible à mesure qu'une probabilité de mouvement indiquée par la valeur statistique d'un pixel correspondant à la carte de valeurs statistiques générée dans la trame précédente est plus élevée ;
l'unité de génération de carte de valeurs statistiques (18) est configurée pour calculer la valeur statistique sur la base d'un masque de pixels qui comprend une pluralité de pixels comprenant chaque pixel de la carte de mouvement cumulative et attribue la valeur statistique calculée à chaque pixel pour générer la carte de valeurs statistiques, la valeur statistique étant une entropie, une variance ou un écart type des valeurs de pixel de la pluralité de pixels dans le masque de pixels.

**2.** Dispositif de traitement d'images selon la revendication 1, dans lequel la carte de différence est obtenue à l'aide d'une image de la trame actuelle et d'une ou plusieurs images de la trame précédente,

dans lequel l'image de trame précédente est une image de sortie de la trame précédente, ou dans lequel l'unité de génération de carte de mouvement cumulative (16) est configurée pour mettre à jour la carte de mouvement cumulative à l'aide de la carte de valeurs statistiques,
dans lequel l'unité de génération d'image de sortie (17) est de préférence configurée pour déterminer un rapport de mélange de l'image de trame actuelle et de l'image de sortie de la trame précédente sur la base de la carte de mouvement cumulative mise à jour.

**3.** Procédé de traitement d'images mis en œuvre par ordinateur pour traiter une image obtenue par imagerie par rayonnement afin de réduire le bruit de manière récursive, consistant à :

recevoir (S31) des images trame par trame obtenues par l'imagerie par rayonnement ;
générer (S34, S35) une carte de mouvement qui inclut des informations de détection de mouvement pour chaque pixel d'une carte de différence obtenue par une différence entre une image de trame actuelle et une image de trame précédente, dans lequel, lors de la génération de la carte de mouvement, la carte de mouvement comportant des informations de détection de mouvement pour chaque pixel est générée par un seuillage pour les valeurs de pixel de chaque pixel de la carte de différence ;

générer (S36) une carte de mouvement cumulative sur la base de la carte de mouvement générée et de la carte de mouvement accumulée jusqu'à une trame précédente ;

générer (S38) une carte de valeurs statistiques formée en attribuant des valeurs statistiques représentant une probabilité de mouvement d'une pluralité de pixels incluant chaque pixel de la carte de mouvement cumulative ; et

mélanger (S37) l'image de l'image de trame actuelle et une image de sortie de la trame précédente sur la base de la carte de mouvement cumulative pour produire une image de sortie de la trame actuelle,

**caractérisé en ce**

**qu'**un seuil de chaque pixel pour le seuillage est défini comme étant plus faible à mesure qu'une probabilité de mouvement indiquée par la valeur statistique d'un pixel correspondant à la carte de valeurs statistiques générée dans la trame précédente est plus élevée,

lors de la génération de la carte de valeurs statistiques, la carte de valeurs statistiques est générée en calculant la valeur statistique sur la base d'un masque de pixels qui comprend une pluralité de pixels comprenant chaque pixel de la carte de mouvement cumulative, puis en attribuant la valeur statistique calculée à chaque pixel,

dans lequel la valeur statistique est une entropie, une variance ou un écart type des valeurs de pixel de la pluralité de pixels du masque de pixels.

4. Procédé de traitement d'image selon la revendication 3, dans lequel la carte de différence est obtenue en utilisant une image de la trame actuelle et une ou plusieurs images des trames précédentes.

5. Procédé de traitement d'image selon la revendication 3, consistant en outre à mettre à jour de la carte de mouvement cumulative à l'aide de la carte de valeurs statistiques,

dans lequel, lors de la génération de l'image de sortie, un rapport de mélange de l'image de trame actuelle et de l'image de sortie de la trame précédente est de préférence déterminé sur la base de la carte de mouvement cumulative mise à jour.

【FIG. 1】

【FIG. 2】

10

| image input unit | ~ 11 |

| difference unit | ~ 14 |

| threshold processing unit | ~ 15 |

| cumulative motion map generation unit | ~ 16 |

| entropy map generation unit | ~ 18 |

| output image generation unit | ~ 17 |

【FIG. 3】

```
┌─────────────────────────┐
│    image acquisition    │──~ S31
└─────────────────────────┘
            │
            ▼
┌─────────────────────────┐
│ difference map generation │──~ S34
└─────────────────────────┘
            │
            ▼
┌─────────────────────────┐
│   motion map generation   │──~ S35
└─────────────────────────┘
            │
            ▼
┌─────────────────────────────┐
│ cumulative motion map generation │──~ S36
└─────────────────────────────┘                  ┌──────────────────────────┐
            │                    ──────────────▶│  entropy map generation  │──~ S38
            ▼                                    └──────────────────────────┘
┌─────────────────────────┐
│      image output       │──~ S37
└─────────────────────────┘
```

【FIG. 4】

(a)

(b)

【FIG. 5】

【FIG. 6】

(a)                    (b)

■ : No Motion
□ : Motion

(c)

【FIG. 7】

motion map
motion map
motion map
motion map

cumulative motion map

100(%)

0(%)

sum

=

【FIG. 8】

【FIG. 9】

(a)                                         (b)

【FIG. 10】

(a)                                         (b)

EP 4 421 729 B1

【FIG. 11】

(a)                              (b)

23

【FIG. 12】

previous
frame image

current
frame image

motion map

image obtained by
removing subject
from background

motion map obtained by
removing noise component

【FIG. 13】

current frame image

previous frame image

difference map

motion map

cumulative motion map

entropy map

updated cumulative motion map (determination of image mixing ratio)

mixing of output image

image output

【FIG. 14】

cumulative motion map

threshold-processed entropy map

updated cumulative motion map

**EP 4 421 729 B1**

**REFERENCES CITED IN THE DESCRIPTION**

**Patent documents cited in the description**

- KR 101432864
- KR 102448069 B1

- JP 6744440 B